# EUROPEAN PATENT APPLICATION

(11) **EP 1 078 632 A1**
(43) Date of publication of application: **28.02.2001**
(21) Application number: 99116026.8
(22) Date of filing: 16.08.1999
(51) Int. Cl.: A61K 31/39, A61K 31/16, A61K 31/35, A61K 31/42, A61K 31/425, A61K 31/44, A61K 31/445, A61K 31/47, A61K 31/50, A61K 31/505, A61K 31/535

(54) **Use of monoamine oxydase inhibitors for the manufacture of drugs intended for the treatment of obesity**

(71) Applicant: SANOFI-SYNTHELABO, 75013 Paris (FR)
(72) Inventor: Rosenweig, Pierre, 75013 Paris (FR)
(74) Representative: Ludwig, Jacques

(57) **Abstract**

The present invention relates to the use of reversible selective inhibitors of monoamine oxydase A (MAO-A), reversible selective inhibitors of monoamine oxydase B (MAO-B) or reversible mixed inhibitors of monoamine oxydase A and B (MAO-A and MAO-B) in the manufacture of drugs intended for the treatment of obesity:
more specifically, the inhibitors are [3(*S*),3a(*S*)]-3-methoxymethyl-7-[4,4,4-trifluorobutoxy]-3,3a,4,5-tetrahydro-1*H*-oxazolo [3,4-a]quinolin-1-one, (*R*)-5-(methoxymethyl)-3-[6-(4,4,4-trifluorobutoxy)benzofuran-3-yl]oxazolidin-2-one and (*R*)-5-methoxymethyl-3-(6-cyclopropylmethoxybenzofuran-3-yl)oxazolidin-2-one, (*S*)-5-methoxymethyl-3-[6-(4,4,4-trifluorobutoxy)-1,2-benzisoxazol-3-yl]oxazolidin-2-one, lazabemide, milacemide, caroxazone, IFO, befloxatone, moclobemide, brofaromine, phenoxathine, esuprone, befol, RS 8359 (Sankyo), T794 (Tanabe), KP 9 (Krenitsky, USA), E 2011 (Eisei), toloxatone, pirlindole, amiflamine, sercloremine and bazinaprine.

The examples give befloxatone.

## Description

The present invention relates to the use of monoamine oxydase inhibitors in the manufacture of drugs intended for the treatment of obesity.

As described in Cheryl P.Kordik and Allen B.Reitz, *J. of Med. Chem.,* Vol 42(2), 181-201, reviewing the various known strategies to treat obesity, obesity is a "chronic condition characterized by overabundance of adipose tissue" which "correlates with risks such as high blood pressure, coronary heart disease, diabetes, altered steroid metabolism, gallstones and certain forms of cancer".

It has now been found that reversible selective inhibitors of monoamine oxydase A (MAO-A), reversible selective inhibitors of monoamine oxydase B (MAO-B) or reversible mixed inhibitors of monoamine oxydase A and B (MAO-B and MAO-A) have activity in decreasing body weight of obese patients.

Accordingly the present invention relates to the use of reversible selective inhibitors of monoamine oxydase A (MAO-A), reversible selective inhibitors of monoamine oxydase B (MAO-B) or reversible mixed inhibitors of monoamine oxydase A and B (MAO-A and MAO-B) for the manufacture of drugs intended for the treatment of obesity.

The invention therefore further relates to a method of treating obesity by administering to a patient in need of such treatment a therapeutically effective amount of a reversible selective inhibitor of monoamine oxydase A, a reversible selective inhibitor of monoamine oxydase B or a reversible mixed inhibitor of monoamine oxydase A and B.

In fact patients who may be treated may be men and women suffering from obesity or overweight.

Among reversible MAO-A inibitors, befloxatone, moclobemide, brofaromine, phenoxathine, esuprone, befol, RS 8359 (Sankyo), T794 (Tanabe), KP 9(Krenitsky, USA), E 2011 (Eisei), toloxatone, pirlindole, amiflamine, sercloremine and bazinaprine may be cited.
These compounds are known and their preparation are described in the art.

Among reversible selective inhibitors of monoamine oxydase B, lazabemide, milacemide, caroxazone and IFO may be cited.

Among reversible selective inhibitors of monoamine oxydase A, reversible selective inhibitors of monoamine oxydase B or reversible mixed inhibitors of monoamine oxydase A and B the following compounds may also be cited:
- compounds disclosed in patent application EP 699680, i.e. 3,3a,4,5-tetrahydro-1*H*-oxazolo[3,4-a]quinolin-1-one derivatives and particularly [3(*S*),3a(*S*)]-3-methoxymethyl-7-(4,4,4-trifluoro-3(*R*)-hydroxybutoxy)-3,3a,4,5-tetrahydro-1*H*-oxazolo[3,4-a]quinolin-1-one and [3(*S*),3a(*S*)]-3-methoxymethyl-7-[4,4,4-trifluorobutoxy]-3,3a,4,5-tetrahydro-1*H*-oxazolo[3,4-a]quinolin-1-one,
- compounds disclosed in patent application WO 96/38444, i.e. oxazolidin-2-one derivatives and particularly (*S*)-5-methoxymethyl-3-[6-(4,4,4-trifluorobutoxy)-1,2-benzisoxazol-3-yl]oxazolidin-2-one,
- compounds disclosed in patent application WO 97/13768, i.e. oxazolidin-2-one derivatives and particularly (*R*)-5-(methoxymethyl)-3-[6-(4,4,4-trifluorobutoxy)benzofuran-3-yl]oxazolidin-2-one and (*R*)-5-methoxymethyl-3-(6-cyclopropylmethoxybenzofuran-3-yl)oxazolidin-2-one.

Befloxatone or 3-[4-(4,4,4-trifluoro-3(*R*)-hydroxybutoxy) phenyl]5(*R*)-methoxymethyl-2-oxazolidinone, which is known for its antidepressive and mild anxiolytic activity is particularly prefered as reversible MAO-A inhibitor. It is a reversible monamine oxydase inhibitor with both a very high affinity for the A isoform (MAO-A) and great selectivity versus the B isoform (MAO-B), which does not affect reuptake of noradrenaline (NA), serotonine (5-HT) or dopamine (DA).
Its chemical synthesis is described in EP 424244.

As reversible MAO-B inhibitor (*S*)-5-methoxymethyl-3-[6-(4,4,4-trifluorobutoxy)-1,2-benzisoxazol-3-yl]oxazolidin -2-one is preferred.

As reversible mixed inhibitor of monoamine oxydase A and B [3(*S*),3a(*S*)]-3-methoxymethyl-7-[4,4,4-trifluorobutoxy]-3,3a,4,5-tetrahydro-1*H*-oxazolo[3,4-a]quinolin-1-one, (*R*)-5-(methoxymethyl)-3-[6-(4,4,4-trifluorobutoxy)benzofuran-3-yl]oxazolidin-2-one and (*R*)-5-methoxymethyl-3-(6-cyclopropylmethoxybenzofuran-3-yl)oxazolidin-2-one are preferred.

The active substance according to the invention can be administred to patients in a variety of pharmaceutical forms well-known in the art and particularly in the form of compositions formulated for administration by the oral, injectable, transdermal or rectal route.
For oral administration, said compositions can take the form of tablets, dragees or capsules prepared by the conventional techniques using known carriers and excipients, such as binding agents, fillers, lubricants and desintegration agents; they can also be in form of solutions, syrups or suspensions.
For administration by the injectable route, the compositions according to the invention may be in the form of injectable solutions, suspensions or emulsions containing an acceptable oily or aqueous liquid carrier.
For transdermal administration, the composition can take the form of a patch wherein the drug can be emcompassed in a gel, solution, ointment or cream.
For rectal administration, the compositions may be in the form of suppositories containing the conventional bases for suppositories.

The percentage of active compound in such compositions may be varied so that a suitable dosage is obtained. The dosage administered to a particular patient is determined by the clinician according to the mode of administration, the age and weight of the patient and the patients response. Unit dosage forms may be administred in a single dose or in multiple divided doses to provide the appropriate daily dosage.

The daily dosage for example of befloxatone can range from about 2.5 to 40 mg, preferably from about 10 to 20 mg.

The following examples relating to pharmacological data and a galenic formulation illustrate the present invention.

### Example 1

### FEEDING BEHAVIOUR IN FASTED RATS

Male Wistar rats (Iffa-Credo) were individually housed in polycarbonate cages (48x26.5x21.5 cm) in a temperature- and humidity-controlled animal colony room (20±2°C) with a 12-hour light dark cycle (7 a.m. - 7 p.m.). At least 1 week before the experiment, every animal was often handled and administered saline by oral route in order to avoid stress. Food and water were available ad libitum, and all testing was done in the home cage. Rats were fasted for 24 hour before testing and allowed free access to water. In the morning of the test day, rats were first assigned to either a treatment or a control group then weighed and administered drug or vehicle p.o. (10.30 a.m.) and returned to their home cage. Thirty minutes later, a measured quantity of food (RMM, Harlan Ibérica) was made available to the animals. The food intake is calculated every hour until 6 hours after the drug administration. (WO95/11894, Gehlert et al., *J. Pharmacol. Exp. Ther.,* 287, 122-127, 1998).
Grams of food consumed by the treated animals every hour was compared to food consumed by the control animals using one-way analysis of variance with a Newman-Keuls' test.

**Table**

| Effect of befloxatone on food consumption during light period (7 a.m.-7 p.m.) in fasted rats (24 hours). Recording and access to food 11 a.m.-2 p.m. | | | | |
|---|---|---|---|---|
| **Group** | **n** | **Food intake (g)** | | |
| | | **0 - 1 hour** | **0 - 2 hours** | **0 - 3 hours** |
| Control (vehicle p.o.) | 5 | 7.56 ± 0.33 | 11.0 ± 1.33 | 11.84 ± 1.37 |
| Befloxatone (3 mg/kg p.o.) | 5 | 5.12 ± 0.78* | 7.94 ± 1.20 | 10.86 ± 0.94 |

| | | | | |
|---|---|---|---|---|
| *p< 0.05 vs control (ANOVA test) | | | | |

### Example 2

### FEEDING BEHAVIOUR IN FED RATS

Male Wistar rats (Iffa-Credo) were individually housed into a temperature- and humidity-controlled animal room (20±2°C) with a 12-hour light dark cycle (4.30 a.m. - 4.30 p.m.). in polycarbonate special cages with transducers connected to MacLab system. This enables to record the food consumption at every moment of day (light/dark phase). A measured quantity of food (RMM, Harlan Ibérica) is placed on the cage just before dark onset.
In order to avoid any kind of stress that could have an effect on their behaviour, every rat is administered saline and put in the cage at least 1 or 2 days before the test. The food consumption is recorded, without interruption, during these days. Once the animal is used to the cage, the test compound or vehicle are administered, by oral route.

Grams of food consumed by the animals during the first 4 hours after drug administration was compared to food consumed by the control animals over the same period of time, using one-way analysis of variance with a Newman-Keuls' test.

**Table**

| Effect of 7 days treatment with befloxatone (10 mg/kg/day, p.o.) on food consumption during dark period (4.30 p.m. - 4.30 a.m.) in fed male Wistar rats | | |
|---|---|---|
| **Days of treatment** | **Food intake (g)** | |
| | **Control vehicle p.o. (n=7)** | **Befloxatone 10 mg/kg/day, p.o. (n=6)** |
| 1 | 3.98 ± 0.63 | 3.03 ± 0.40 |
| 2 | 5.91 ± 0.85 | 3.31 ± 1.00 |
| 3 | 7.95 ± 0.68 | 5.32 ± 0.69* |
| 4 | 7.35 ± 0.57 | 6.12 ± 0.50 |
| 5 | 8.75 ± 0.76 | 6.10 ± 0.59* |
| 6 | 9.69 ± 0.98 | 6.98 ± 0.61* |
| 7 | 10.1 ± 0.74 | 6.95 ± 0.75* |

| | | |
|---|---|---|
| *p< 0.05 vs control (ANOVA test) | | |

These results show that in the model using **fasted rats**, befloxatone (3 mg/kg p.o.) inhibits food intake by about 25% during the first hour after administration of the drug, and in the model of **fed rats** with recording of food consumption in the dark, befloxatone (10 mg/kg p.o.), once a day for 7 days, inhibits as from the third day, food intake during the first four hours after drug administration.

### Example 3

### ORAL FORMULATION

| | | |
|---|---|---|
| Befloxatone | 2.5 mg | 0.125 kg |
| Maize starch | 5 mg | 0.250 kg |
| Lactose monohydrate | 83 mg | 4.150 kg |
| Povidone K29/32 | 5 mg | 0.250 kg |
| Crospovidone | 4 mg | 0.200 kg |
| Magnesium stearate | 0.5 % | 0.025 kg |
| size 3 gelatine capsule | | |

The befloxatone and approximately 10% of the lactose (415 g), were premixed for 10 minutes using a Turbula mixer. The mixture was then transferred to a Diosna mixer-granulator. The remainder of the lactose, the maize starch, the povidone, and half the crospovidone were added and mixed for 3 minutes. A sufficient quantity of water was added (13%) and the mixture granulated for 3 minutes. The granulate was dried in a ventilated oven and calibrated at 0.63 mm. The rest of the crospovidone, plus the magnesium stearate was added to the resulting granulate, and the whole was mixed using a Turbula mixer for 10 minutes, and then filled into size 3 capsules to a unit mass of 100 mg.

## Claims

1. Use of a reversible selective inhibitor of monoamine oxydase A, reversible selective inhibitor of monoamine oxydase B or a reversible mixed inhibitor of monoamine oxydase A and B for the manufacture of a medicament intended for the treatment of obesity.

2. Use of a reverible mixed inhibitor of monoamine oxydase A and B according to claim 1.

3. The use according to claim 2 wherein the reversible mixed inhibitor of monoamine oxydase A and B is chosen among [3(*S*),3a(*S*)]-3-methoxymethyl-7-[4,4,4-trifluorobutoxy]-3,3a,4,5-tetrahydro-1*H*-oxazolo[3,4-a]quinolin-1-one, (*R*)-5-(methoxymethyl)-3-[6-(4,4,4-trifluorobutoxy)benzofuran-3-yl]oxazolidin-2-one and (*R*)-5-methoxymethyl-3-(6-cyclopropylmethoxybenzofuran-3-yl)oxazolidin-2-one.

4. Use of a reversible selective inhibitor of monoamine oxydase B according to claim 1.

5. The use according to claim 4 wherein the reversible selective monoamine oxydase B is chosen among lazabemide, milacemide, caroxazone and IFO.

6. The use according to claim 4 wherein the reverible selective monoamine oxydase B is (*S*)-5-methoxymethyl-3-[6-(4,4,4-trifluorobutoxy)-1,2-benzisoxazol-3-yl]oxazolidin-2-one.

7. Use of a reversible selective inihibtor of monoamine oxydase A according to claim 1.

8. The use according to claim 7 wherein the reversible selective inhibitor of monoamine oxydase A is chosen among befloxatone, moclobemide, brofaromine, phenoxathine, esuprone, befol, RS 8359 (Sankyo), T794 (Tanabe), KP 9 (Krenitsky, USA), E 2011 (Eisei), toloxatone, pirlindole, amiflamine, sercloremine and bazinaprine.

9. The use according to claim 7 wherein the reversible selective inhibitor of monoamine oxydase A is befloxatone.

10. The use according to claim 9 wherein the dosage amount of befloxatone is from about 2.5 to 40 mg per day.

11. The use according to claim 10 wherein the amount of befloxatone to be administred is from 10 to 20 mg.

12. The use according to any of claims 1 to 11 wherein the inhibitor of monoamine oxydase is intended for administration by the oral, injectable, transdermal or rectal route.
